(19) Europäisches Patentamt
European Patent Office
Office européen des brevets

(11) **EP 3 378 848 B1**

(12) **EUROPÄISCHE PATENTSCHRIFT**

(45) Veröffentlichungstag und Bekanntmachung des Hinweises auf die Patenterteilung:
**24.11.2021  Patentblatt 2021/47**

(51) Int Cl.:
*C07C 1/02* (2006.01)          *C07C 9/04* (2006.01)
*C07C 29/159* (2006.01)          *C07C 31/04* (2006.01)
*B01J 8/22* (2006.01)

(21) Anmeldenummer: **17162561.9**

(22) Anmeldetag: **23.03.2017**

(54) **HYDRIERUNGSVERFAHREN ZUR SYNTHESE VON METHAN UND METHANOL**
HYDROGENATION METHOD FOR SYNTHESIS OF METHANE AND METHANOL
PROCÉDÉ D'HYDROGÉNATION DESTINÉ À LA SYNTHÈSE DE MÉTHANE ET DE MÉTHANOL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**26.09.2018  Patentblatt 2018/39**

(73) Patentinhaber: **Karlsruher Institut für Technologie**
**76344 Eggenstein-Leopoldshafen (DE)**

(72) Erfinder:
• **Lefebvre, Jonathan**
**76135 Karlsruhe (DE)**
• **Ortloff, Felix**
**77836 Rheinmünster (DE)**
• **Müller, Christian**
**76137 Karlsruhe (DE)**
• **Bajohr, Siegfried**
**69168 Wiesloch (DE)**

• **Graf, Frank**
**76131 Karlsruhe (DE)**
• **Kolb, Thomas**
**67480 Edenkoben (DE)**

(74) Vertreter: **Gille Hrabal Partnerschaftsgesellschaft mbB**
**Patentanwälte**
**Brucknerstraße 20**
**40593 Düsseldorf (DE)**

(56) Entgegenhaltungen:
**CN-A- 104 549 334**

• **Jonathan Lefebvre ET AL: "Improvement of three-phase methanation reactor performance for steady-state and transient operation", FUEL PROCESSING TECHNOLOGY., vol. 132, 13 January 2015 (2015-01-13), pages 83-90, XP055405926, NL ISSN: 0378-3820, DOI: 10.1016/j.fuproc.2014.10.040**

**Beschreibung**

**EINLEITUNG**

Die vorliegende Erfindung betrifft ein neues Hydrierungsverfahren zur Synthese von Methan und Methanol durch Hydrierung von kohlenstoffhaltigen Gasen mit Wasserstoff unter Verwendung hydrierter Wärmeträgeröle, sowie die Verwendung eines Reaktorsystems zur Durchführung des Verfahrens.

**HINTERGRUND**

Die vorliegende Erfindung betrifft den Bereich der chemisch-katalytischen Umsetzung von kohlenstoffhaltigen Gasen mit Wasserstoff ($H_2$) zur Erzeugung von Methan (SNG, Substitute Natural Gas oder Synthetic Natural Gas bzw. synthetisches Erdgas) und Methanol, sowie ein neues Verfahren mit optimierter Prozessführung von Anlagen zur Erzeugung von Methan und Methanol. Darin umfassen kohlenstoffhaltige Gase Kohlenstoffoxide, wie beispielsweise CO, $CO_2$. u.ä.. Dabei kann CO beispielsweise aus Synthesegas (z.B. aus der Biomassevergasung etc.) stammen, $CO_2$ z.B. aus Abgasströmen oder aus der Luft kann direkt als C-Quelle für die Hydrierungsreaktionen genutzt werden.

SNG ist ein Erdgassubstitut, das z.B. auf der Basis von Kohle, vor allem Braunkohle, oder Biomasse (Bio-SNG bzw. Biomethan) über Synthesegas hergestellt werden kann. SNG auf der Basis von Braunkohle wird über eine Kohlevergasung und auf der Basis von Biomasse über eine Biomassevergasung zu Synthesegas produziert. In beiden Fällen wird das entstehende Synthesegas nach einer Reinigung von Partikeln, Kohlendioxidanteilen und Schwefel- und Chlorverbindungen einer anschließenden Methanisierung zugeführt. Um Erdgas zur Rohstoffversorgung und für die Energieerzeugung ersetzen zu können, muss SNG in das Erdgasnetz eingespeist werden können und daher Erdgas in seiner Zusammensetzung und seinen Eigenschaften möglichst weitgehend entsprechen.

Ein besonderer Fokus wird heute verstärkt auf sogenannte Power-to-X (PtX) Prozesse gelegt, worin aus fluktuierend anfallender erneuerbarer Energie (z. B. aus Solar- und Windkraft) Wasserstoff erzeugt wird, welcher anschließend in Hydrierreaktionen mit Kohlenstoffoxiden wie beispielsweise CO, $CO_2$ u.ä. zu chemischen Energieträgern umgesetzt wird. Ziel von PtX-Prozessen ist die mittel- und langfristige Speicherung von überschüssiger elektrischer Energie hinsichtlich einer zeitlichen und räumlichen Entkopplung ihrer Erzeugung und Nutzung, bzw. der Sektor übergreifenden Verwendung, z.B. in Form von CNG (Compressed Natural Gas, komprimiertes Erdgas), LNG (Liquefied Natural Gas, Flüssigerdgas), DME (aus Biomasse hergestellter Dimethylether) oder Fischer-Tropsch Produkten (Fischer-Tropsch-Kraftstoffen) im Verkehrssektor oder z. B. in Form von Methanol, Ameisensäure etc. im Chemiesektor, etc.. So beschreibt beispielsweise Wikipedia unter dem Begriff "Chemische Wasserstoffspeicher" Medien zur Speicherung und zum Transport von Wasserstoff, worin solche Speichermedien an einem "energiereichen" Ort erzeugte Energie nutzen, um die energiearme Form des Trägerstoffs in einer chemischen Reaktion mit Energie zu beladen, wodurch das mit Wasserstoff beladene Medium dann verlustfrei gelagert, transportiert und verteilt werden kann. Außerdem werden dort auch Ansätze zur chemischen "Wasserstoffspeicherung im weiteren Sinne" erwähnt, worin Wasserstoffspeicher (z.B. in Form von Methan, Methanol etc.) so eingesetzt werden, dass daraus in der Nutzungsphase (Umsetzung) der Wasserstoff nicht zurückgewonnen wird, sondern der "Wasserstoffspeicher" (z.B. Methan, Methanol etc.) in einer chemischen Reaktion umgesetzt (verbrannt) wird. Der "Träger" des Wasserstoffs wird bei all diesen Ansätzen nicht recycelt. Als Beispiele für mögliche Konversionsmethoden werden aufgeführt:

Die Herstellung von Methan aus $H_2$ und $CO_2$ (vergl. Sabatier-Prozess, Windgas)
Die Herstellung von Methanol aus $H_2$ und $CO_2$ bzw. CO
Die Herstellung von Ammoniak aus $H_2$ und $N_2$ (vergl. Haber-Bosch-Verfahren)
Die Hydrierung von Kohle (Bergius-Verfahren oder Fischer-Tropsch-Verfahren)

Dadurch dass bei der Umsetzung der "Wasserstoffspeicher" (z.B Methan, Methanol, Ammoniak etc.), wie sie aus diesen Konversionsmethoden gewonnen werden, kein elementarer Wasserstoff zurückgewonnen wird, weicht darin die Art der Nutzung der gespeicherten Energie (Wasserstoff) von der oben beschriebenen Wasserstoffspeicherung im engeren Sinne ab.

Bei Hydrierreaktionen, wie der Methanisierung, stellt das das Wärmemanagement im Synthesereaktor ein großes Problem dar. Hydrierreaktionen sind stark exotherme Reaktionen:

$$CO + 3\,H_2 \rightleftarrows CH4 + H_2O \qquad \Delta_R H^0 = -206\,\frac{kJ}{mol}$$

(fortgesetzt)

$$CO_2 + 4\,H_2 \rightleftarrows CH_4 + 2\,H_2O \qquad \Delta_R H^0 = -165\,\frac{kJ}{mol}$$

$$CO_2 + H_2 \rightleftarrows CO + H_2O \qquad \Delta_R H^0 = 41\,\frac{kJ}{mol}$$

Exotherme Reaktionen sind dadurch charakterisiert, dass ab bestimmten Temperaturen eine thermodynamisch bedingte Limitierung des Umsatzes resultiert. Sollen jedoch in nur einer Reaktorstufe hohe Umsatze erzielt werden, so muss das Temperaturprofil innerhalb des Reaktors optimiert, bzw. die Warme effizient aus dem Reaktorsystem ausgetragen werden können.

## STAND DER TECHNIK

Der Übersichtsartikel von Götz et al. "Renewable Power-to-Gas: A technological and economic review"; Renewable Energy 85, S. 1371-1390 beschreibt bisher bekannte Verfahren der Methanisierung.

Die mit der Verwendung konventioneller Festbettreaktoren verbundenen Nachteile liegen in der erschwerten Abfuhr der in der exothermen Reaktion anfallenden Wärme über die Reaktorwand an das Wärmeträgermedium aufgrund der schlechten Wärmeleitung und der hohen Energiefreisetzungsdichte im Festbettreaktor. Die Verwendung der in solchen Festbettreaktoren üblicherweise verwendeten Katalysatorpellets bewirkt außerdem einen vergleichsweise hohen Druckverlust.

Aus der DE 102011009163 A1 ist ein Verfahren der Dreiphasen-Methanisierung von CO- oder $CO_2$- und $H_2$-haltigem Eduktgas unter Verwendung eines Blasensäulenreaktors, der als Flüssigphase mit ionischen Flüssigkeiten beschickt ist, worin der Katalysator in Partikelform als Festphase suspendiert ist, und worin die Edukt- und Produktgase gasförmig vorliegen. Darin kann durch Einsatz des darin beschriebenen Blasensäulenreaktors zwar die in der Hydrierreaktion von CO oder $CO_2$ zu Methan anfallende Wärme effizient aus dem System entfernt werden, es besteht jedoch der Nachteil einer starken Limitierung des Umsatzes durch den zuvor erforderlichen Übergang des gasförmig zugeführten Wasserstoffs aus der Gasphase in die flüssige Phase.

Aus der CN 104645898 (A) ist ein Verfahren zur Methanisierung von Synthesegas unter Verwendung eines sogenannten Rieselbett-Reaktors (Trickle-Bed-Reaktor) bekannt, worin ein mit einem inerten Wärmeträgeröl gefluteter Festbettreaktor verwendet wird. Der darin beschriebene Rieselbett-Reaktor (Trickle-Bed-Reaktor) wird im Gleichstromverfahren unter Verwendung von kommerziellen Katalysatorpellets betrieben. Das verwendete Wärmeträgermedium nimmt die Reaktionswärme im Reaktor ebenso effizient auf und gibt die Wärme über einen externen Wärmeaustauscher wieder ab. Auch in dem hier beschriebenen Methanisierungsverfahren liegen die Edukt- und Produktgase gasförmig vor, was mit den oben genannten Nachteilen der Stofftransportlimitierung verbunden ist. Die Verwendung von kommerziellen Katalysatorpellets führt dazu, dass die Katalysatorschüttung bereits bei vergleichsweise geringen Flüssigkeitsbelastungen geflutet wird.

Insgesamt basiert der vorgenannte Stand der Technik auf der Idee das Wärmemanagement der exothermen Hydrierungsreaktion zu optimieren, um eine bessere Reaktoreffizienz im Vergleich zu konventionellen Reaktoren, wie beispielsweise einer Kaskade von adiabaten Festbetten, zu erzielen und die Langzeitstabilität des Katalysators zu erhöhen.

Der vorgenannte Stand der Technik berücksichtigt jedoch nicht die geringe Löslichkeit des eingesetzten gasförmigen Wasserstoffs bei den angestrebten Reaktionsbedingungen. Auch ist zu berücksichtigen, dass in den beschriebenen Verfahren der Stofftransport der Reaktionsgase durch die Flüssigphase die Reaktion hemmt. Der Druckverlust über einem Blasensäulenreaktor ist ebenfalls nicht zu vernachlässigen und spielt vor allem beim gefluteten Festbett eine erhebliche Rolle. Durch den Überschuss an Flüssigphase bei der Dreiphasen-Methanisierung wird der Reaktor quasi isotherm betrieben, was Nachteile bezüglich der Reaktionsgeschwindigkeit bewirkt. Da im isothermen Betrieb die Temperatur im Reaktor quasi überall gleich ist, kann kein geeignetes Temperaturprofil eingestellt werden.Ein Gleichstrombetrieb eines Rieslebetts (Trickle-Bed) bewirkt einen Anstieg der Temperatur in Richtung des Reaktorausgangs, was sich nachteilig auf die Lage des chemischen Gleichgewichts und somit auf die Reaktionsumsätze auswirkt. Bei Verwendung von Blasensäulenreaktoren ist eine axiale Rückvermischung der Reaktionsphase nicht auszuschließen, was ebenfalls eine verringerte Reaktoreffizienz mit sich bringt.

CN 104549334 A offenbart ein Verfahren zur Synthese von Methan, bei dem in einem Reaktor ein "Hydrogen supply solvent" wie Tetrahydronaphthalin oder Decalin und ein Katalysator vorgelegt wird und anschließend Wasserstoff und Kohlenmonoxid gasförmig eingeleitet werden.

## AUFGABENSTELLUNG

Die Aufgabe der vorliegenden Erfindung bestand darin, ein verbessertes Hydrierungsverfahren zur Synthese von Methan und/oder Methanol aus kohlenstoffhaltigen Gasen und Wasserstoff bereitzustellen, welches die Nachteile der bekannten Verfahren vermeidet. Insbesondere sollte sich das verbesserte Hydrierungsverfahren durch eine optimierte Prozessführung der Syntheseanlagen auszeichnen. Außerdem sollte die Problematik der Gleichgewichtslimitierung in adiabaten Festbettreaktoren gelöst und eine zuverlässige Einstellung des Gleichgewichts der Hydrierungsreaktion ermöglicht werden. Damit bestand in einem weiteren Aspekt die Aufgabe insbesondere auch darin, die Reaktionsumsätze gegenüber herkömmlichen Dreiphasensystemen zu verbessern. In einem weiteren Aspekt bestand die Aufgabe auch darin, eine verbesserte Lösung zur Abführung der in der exothermen Reaktion im Reaktor anfallenden Wärme bereitzustellen und eine effiziente Wärmekontrolle zu ermöglichen. In einem weiteren Aspekt sollte außerdem der Druckverlust über die Reaktorlänge verringert werden. In einem zusätzlichen Aspekt bestand die Aufgabe außerdem darin, einen verbesserten Reaktor zur Durchführung eines solchen verbesserten Verfahrens bereitzustellen.

## DETAILLIERTE BESCHREIBUNG DER ERFINDUNG

Die vorliegende Erfindung löst die gestellte Aufgabe durch Bereitstellung eines neuen Verfahrens zur Synthese von Methan ($CH_4$; SNG, Substitute Natural Gas) und/ oder Methanol ($CH_3OH$) durch Hydrierung von CO und/oder $CO_2$ mit Wasserstoff, umfassend die folgenden Verfahrensschritte:

a) Bereitstellen eines Reaktorsystems mit

a-a) einem Dreiphasenreaktor, der im Gegenstromverfahren betrieben wird, sowie
a-b) einem Reaktor zur Hydrierung eines Wärmeträgermediums und gegebenenfalls einem Speicher oder Vorratsbehälter für ein hydriertes Wärmeträgermedium (a-c) und/oder gegebenenfalls einem Speicher oder Vorratsbehälter für ein unhydriertes Wärmeträgermedium (a-d);

b) Beschicken des Dreiphasenreaktors (a-a) mit einer katalytischen Füllung;
c-a) Hydrierung des Wärmeträgermediums in dem Reaktor a-b),
c) kontinuierliches Einleiten des hydrierten Wärmeträgermediums in den Dreiphasenreaktor (a-a) in Form einer flüssigen Phase, worin der Wasserstoff chemisch gebunden an das Wärmeträgermedium vorliegt;
d) Einleiten von CO und/oder $CO_2$ in den Dreiphasenreaktor (a-a); und
e) Abführen der umgesetzten Syntheseprodukte.

Die Erfinder der vorliegenden Erfindung fanden überraschend, dass es möglich ist, durch die Verwendung eines hydrierten Wärmeträgeröls in Schritt c) das Problem der Stofftransportlimitierung in konventionellen Dreiphasensystemen zu lösen, aber dennoch eine gute Temperaturkontrolle durch eine weiterhin eingesetzte in-situ Kühlung des Katalysators mit dem Wasserstoffbeladenen hochleitfähigen Wärmeträgermedium zu realisieren. Die Einführung der zusätzlichen Flüssigphase mit hoher Wärmekapazität in das Reaktorsystem sorgt bei dynamischer Fahrweise des Reaktors für ein stabiles Reaktorverhalten. Statt den Wasserstoff wie in dem beispielsweise aus der oben genannten DE 102011009163 A1 oder CN 104645898 (A) bekannten Verfahren aus der Gasphase in das Reaktionssystem einzubringen, was die oben dargestellten Nachteile mit sich bringt, zeichnet sich das erfindungsgemäße Verfahren insbesondere dadurch aus, dass der Wasserstoff in Form einer flüssigen Phase durch chemische Bindung an das (flüssige) Wärmeträgermedium in den Reaktor eingeleitet wird. Der Wasserstoff kann dadurch in erheblich größerer Konzentration - in chemisch gebundener Form zusammen mit dem Wärmeträgermedium - an die Katalysatoroberfläche eingebracht werden. Das dem erfindungsgemäßen Verfahren zugrundeliegende Hydrierungsprinzip kann auch als Transferhydrierung bezeichnet werden.

Die vorliegende Erfindung zeichnet sich somit insbesondere durch die Verwendung eines hydrierten Wärmeträgeröls als Reduktionsmittel in der Hydrierungsreaktion aus. Dies ermöglicht eine Steigerung der Reaktionsgeschwindigkeit, da gasförmiger Wasserstoff nicht erst in der Flüssigphase gelöst werden muss, sondern in gebundener (und darüber flüssiger) Form direkt zum Katalysator geführt werden kann.

Als Wärmeträgermedien eignen sich im Prinzip alle unter den Reaktor- und Reaktionsbedingungen für die chemisch-katalytische Synthese von Methan und Methanol reversibel hydrierbaren Wärmeträgermedien. Insbesondere geeignet sind hierfür reversibel hydrierbare Wärmeträgeröle. Hydrierbare Wärmeträgermedien und deren Verwendung als Wasserstoffspeichermedien beschreibt beispielsweise die WO 2014/082801. Derartige Wärmeträgermedien können als bei Raumtemperatur flüssige Mischung aus zwei oder mehreren Verbindungen, die auf den Elementen Kohlenstoff und Wasserstoff basierend aufgebaut sind und in einzelnen bekannten Zusammensetzungen ein synthetisches Substanzgemisch bilden, das als Wärmeträgerflüssigkeit einsetzbar ist, und die dadurch gekennzeichnet ist, dass die Mischung mindestens eine Verbindung mit mindestens zwei nicht-kondensierten, nicht pi-konjugierten aromatischen Einheiten enthält und in katalytischen Verfahren zur Bindung bzw. Freisetzung von Wasserstoff an die bzw. von der Mischung verwendet werden kann, definiert werden. Insbesondere umfasst die vorliegende Erfindung solche Wärmeträgermedien, wie in der WO 2014/082801 beschrieben.

Auch die EP 1475349 A2 beschreibt geeignete reversibel hydrierbare Wärmeträgermedien und deren Verwendung als Wasserstoffspeicher.

Sowohl die WO 2014/082801 als auch die EP 1475349 A2 offenbart allenfalls die reversible Hydrierung der darin beschriebenen Wärmeträgermedien, sowie die Verwendung dieser als Speichermedium für Wasserstoff (im klassischen Sinne). Das Prinzip der chemischen Wasserstoffspeicher (im klassischen Sinne) ist, an einem "energiereichen" Ort erzeugte Energie zu nutzen, um die energiearme Form des Trägerstoffs in einer chemischen Reaktion mit Energie, z.B. elektrolytisch hergestelltem Wasserstoff, zu beladen. Durch die Anreicherung mit Wasserstoff speichert das Medium dessen chemische Energie. Dieses mit Wasserstoff angereicherte Material kann verlustfrei über große Zeiträume gelagert, mit hoher Energiedichte transportiert und verteilt werden. Am Ort und zur Zeit des Energiebedarfs wird die energiereiche Form unter Freisetzung von Wasserstoff wieder energetisch entladen und zum Ort der Energieerzeugung zurückgebracht. Dort steht sie zur erneuten Energieaufnahme bereit. Damit werden diese chemischen Wasserstoffspeicher zum Auffangen von Leistungsschwankungen bei der Energieerzeugung eingesetzt, indem die ursprünglich als elektrischer Strom vorliegende Energie gespeichert, transportiert und z.B. in Verbindung mit Brennstoffzellen wieder in Strom rückverwandelt wird. Die Verwendung derartiger hydrierter Wärmespeichermedien als flüssige Form der Wasserstoffquelle in der Synthese von Methan, Methanol u.a. durch Hydrierung von kohlenstoffhaltigen Gasen wie CO, $CO_2$, Synthesegas etc. ist aus dem Stand der Technik nicht bekannt.

Das erfindungsgemäß verwendete reversibel hydrierbare Wärmeträgermedium ist bevorzugt ausgewählt aus der Gruppe der sogenannten LOHC (Liquid Organic Hydrogen Carrier), die bis dato ausschließlich als Wasserstoffspeicher zum Transport von Energie wie vorstehend beschrieben genutzt werden. Das Prinzip kann wie folgt beschrieben werden:

$$LOHC\text{-}H_0 + \tfrac{1}{2}\, n\, H_2 \rightleftarrows LOHC\text{-}H_n \;(H_2 \text{ - Speicherung})$$

$$LOHC\text{-}H_n \rightleftarrows LOHC\text{-}H_0 + \tfrac{1}{2}\, n\, H_2 \;(H_2 \text{ - Freisetzung})$$

Darin bezeichne $LOHC\text{-}H_0$ ein LOHC ohne H-Anlagerung (unhydriert) und $LOHC\text{-}H_n$ ein LOHC mit n Wasserstoff (hydriert), wobei n einen ganzzahligen Wert von $\geq 1$ darstellt.

Die $H_2$-Speicherung läuft bei hohen $H_2$-Partialdrücken und moderaten Temperaturen, während die $H_2$-Freisetzung bei 1 atm und hohen Temperaturen betrieben wird. Daher liegt $H_2$ als Produkt bei 1 atm vor und muss für industrielle Anwendungen wieder komprimiert werden.

Im Gegensatz dazu lässt sich der Vorgang der reversiblen Wärmeträgermedien-Hydrierung in der erfindungsgemäßen Verwendung, wie folgt beschreiben:

$$LOHC\text{-}H_0 + \tfrac{1}{2}\, n\, H_2 \rightleftarrows LOHC\text{-}H_n \;(H_2 \text{ - Speicherung})$$

$$LOHC\text{-}H_n + x\, CO \rightleftarrows LOHC\text{-}H_{(n-y)} + C_xH_yO_x \;(CO\text{-Hydrierung})$$

bzw.

$$LOHC\text{-}H_n + x\, CO_2 \rightleftarrows LOHC\text{-}H_{(n-y)} + C_xH_yO_{2x} \;(CO_2\text{-Hydrierung})$$

Damit ergibt sich beispielsweise für Methanisierungsreaktionen gemäß der vorliegenden Erfindung:

$$LOHC\text{-}H_n + CO \rightleftarrows LOHC\text{-}H_{(n-6)} + CH_4 + H_2O$$

bzw.

$$LOHC\text{-}H_n + CO_2 \rightleftarrows LOHC\text{-}H_{(n-8)} + CH_4 + 2\, H_2O$$

Im Gegensatz zu der bekannten Verwendung als Wasserstoffspeichermedium nehmen bei der erfindungsgemäßen Verwendung die LOHC-Wärmeträgermedien an der $CO/CO_2$-Hydrierung teil und sind nicht mehr nur inerte Wärmeträgerflüssigkeiten. Das Zielprodukt (Syntheseprodukt) ist das zu synthetisierende Methan bzw. Methanol statt molekularer Wasserstoff ($H_2$). Da die $CO_2$-Hydrierung unter Druck erfolgen kann, ist das Produkt bereits komprimiert. Im Vergleich zu herkömmlichen Dreiphasenreaktoren muss in dem erfindungsgemäßen Verfahren kein $H_2$ von der

Gasphase in die Flüssigkeiten überragen werden, ein physikalisches Lösen des gasförmigen $H_2$ entfällt.

Erfindungsgemäß geeignet sind insbesondere solche LOHC, die unter den Reaktor- und Reaktionsbedingungen für die chemisch-katalytische Synthese von Methan und/oder Methanol reversibel hydrierbar sind. Des Weiteren sollten sich die erfindungsgemäß verwendeten LOHC durch eine oder mehrere der folgenden Eigenschaften auszeichnen:

- flüssiger Zustand im gesamten relevanten Temperaturbereich,
- hohe $H_2$-Speicherkapazität,
- hohe $CO/CO_2$-Löslichkeit,
- hohe Temperatur- und Zyklenstabilität,
- niedriger Dampfdruck,
- günstig,
- industriell verfügbar.

Wärmeträgermedien aus der Gruppe der LOHC (Liquid Organic Hydrogen Carrier) umfassen insbesondere die in der WO 2014/082801 und in der EP 1475349 A2 offenbarten, wie insbesondere Cykloalkene wie Dibenzyltoluol (Marlotherm®), Benzol, Toluol, Naphthalin, Biphenyl; Cykloalkane; Thiophen wie Methylthiophen; Carbazol wie N-Ethylcarbazol; Indol wie 2-Methylindol, etc .

Erfindungsgemäß wird besonders bevorzugt LOHC-$H_n$ eingesetzt (worin n für einen ganzzahligen Wert von mindestens ($\geq$) 1 steht). Dazu wird beispielsweise in Schritt c) des erfindungsgemäßen Verfahrens Wasserstoff in Form eines hydrierten Wärmeträgermediums LOHC-$H_n$ (worin n für einen ganzzahligen Wert von mindestens ($\geq$) 1 steht) in den Dreiphasenreaktor a-a) eingeleitet. Bevorzugt wird ein LOHC-$H_n$ mit $n \geq 4$ eingesetzt. Ganz besonders bevorzugt wird ein LOHC aus der oben genannten Gruppe eingesetzt. Ganz besonders bevorzugt wird Marlotherm® (Dibenzyltoluol) verwendet.

Erfindungsgemäß erfolgt die chemische Bindung des Wasserstoffs an das Wärmeträgermedium, wie vorstehend definiert, in einem Verfahrensschritt der Vorhydrierung.

Dazu umfasst das erfindungsgemäße Reaktorsystem a) neben dem Dreiphasenreaktor a-a), außerdem einen integrierten oder vorgelagerten Reaktor a-b) zur Hydrierung des Wärmeträgermediums, sowie gegebenenfalls einen (oder mehrere) Speicher oder Vorratsbehälter a-c) für das (vor-)hydrierte Wärmeträgermedium.

Der erfindungsgemäße Dreiphasenreaktor a-a) wird bevorzugt ausgewählt aus der Gruppe umfassend Blasensäulenreaktoren und Rieselbettreaktoren (Trickle-Bed-Reaktor). Erfindungsgemäß besonders bevorzugt sind Rieselbettreaktoren (Trickle-Bed-Reaktoren). Bei Rieselbettreaktoren wird eine flüssige Phase am Reaktorkopf aufgegeben und rieselt über die Katalysatorschüttung, während das eingeleitete Gas dazu im Gleich- oder Gegenstrom geführt wird, wie z.B. von Hagen J. in "Chemiereaktoren: Auslegung und Simulation", John Wiley & Sons, Seite 10-10, 2012, beschrieben. Grundsätzlich sind alle herkömmlichen Rieselbettreaktoren geeignet, insbesondere, wenn diese im Gegenstromverfahren betrieben werden können, wie beispielsweise solche wie von Dudukovic et al. in "Three-Phase Trickle-Bed Reactors", Ullmann's Encyclopedia of Industrial Chemistry, Seite 4, 2013 beschrieben, und die von der vorliegenden Erfindung umfasst sind.

Hinsichtlich der möglichen Ausgestaltung eines Blasensäulenreaktors wird auf die oben genannte DE 102011009163 A1 verwiesen.

In dem erfindungsgemäßen Verfahren wird der Synthesereaktor im Gegenstromverfahren betrieben. Dadurch können die Temperatur- und Konzentrationsprofile im Synthesereaktor optimiert werden. Dies ist besonders bei stark gleichgewichtslimitierten Reaktionen wie der Methanisierung vorteilhaft, da eine genaue Temperaturkontrolle am Austritt des Synthesereaktors über die Zulauftemperatur der Flüssigkeit gegeben ist. Dies kann insbesondere z.B. durch Ausführung des Synthesereaktors in Form eines Rieselbettreaktors (Trickle-Bed) realisiert werden, welcher bevorzugt ist.

In dem erfindungsgemäßen Verfahren ist die katalytische Füllung zur Beschickung des Dreiphasenreaktors a-a) in Schritt b) nicht weiter hinsichtlich ihrer Form beschränkt, solange sie zur Umsetzung der chemisch-katalytischen Reaktion geeignet ist. Bevorzugt wird die katalytische Füllung ausgewählt aus der Gruppe umfassend Festkörperkatalysatoren, partikelförmige Katalysatoren, pulverförmige Katalysatoren, sowie Katalysatoren in Form katalytisch beschichteter Füllkörperpartikel. Katalytisch beschichtete Füllkörperpartikel (katalytisch beschichtete poröse Füllkörperpartikel) sind erfindungsgemäß bevorzugt. Geeignete poröse Füllkörperpartikel sind prinzipiell bekannt. Der Fachmann weiß, dass die Füllkörpergröße mit dem Reaktordurchmesser skaliert wird, welcher letztlich von der Leistungsklasse der Syntheseanlage festgelegt wird und ist in der Lage, geeignete Füllkörperpartikel mit geeigneter Partikelgröße auszuwählen.

Die Verwendung von katalytisch beschichteten Füllkörpern oder katalytisch beschichteten Füllkörperpartikeln ermöglicht einen Einsatz hydrodynamisch optimierter katalytischer Füllkörperpackungen, wodurch der Druckverlust über die Reaktorlänge minimiert werden kann.

Als Katalysator können in dem erfindungsgemäßen Verfahren solche ausgewählt werden, die üblicherweise zur Hydrierung von kohlenstoffhaltigen Gasen eingesetzt werden, wie z.B. Katalysatoren auf Basis von Metallen ausgewählt aus Fe, Co, Ni, Cu, Ru, Rh, Pd, Ag, Os, Ir, Pt, Au und Mo, sowie Kombinationen davon. Der Katalysator kann geträgert oder ungeträgert verwendet werden, wobei bevorzugte Trägermaterialien ausgewählt werden aus $Al_2O_3$, $SiO_2$, MgO und ZnO, sowie Kombinationen davon. Erfindungsgemäß werden besonders bevorzugt Katalysatoren auf Basis von Ni und Cu ausgewählt, wie insbesondere Katalysatoren auf Basis von NiO und Cu-ZnO-$Al_2O_3$ Systeme. Dabei werden Katalysatoren auf Ni-Basis, wie insbesondere NiO, besonders bevorzugt in der Synthese von Methan gemäß dem erfindungsgemäßen Verfahren eingesetzt. Für die Synthese von Methanol gemäß dem erfindungsgemäßen Verfahren werden besonders bevorzugt Katalysatoren auf Cu-Basis, wie insbesondere Cu-ZnO-$Al_2O_3$ Systeme, eingesetzt.

Die in dem erfindungsgemäßen Verfahren eingesetzten kohlenstoffhaltigen Gase sind CO und $CO_2$. Dabei können die kohlenstoffhaltigen Gase thermochemisch aus Biomasse hergestellt werden, z.B. in einem dem erfindungsgemäßen Reaktorsystem vorgeschalteten Reaktor zur thermochemischen Vergasung von Biomasse, wie in der oben genannten DE 102011009163 A1 beschrieben.

In dem erfindungsgemäßen Verfahren wird bevorzugt ein Verhältnis von Kohlenstoffoxiden (CO und/oder $CO_2$) zu Wasserstoff ($H_2$) von 1:2 bis 1:6, bevorzugter 1:3 bis 1:5, noch bevorzugter 1:3 oder 1:4 eingestellt. In der Methanolsynthese wird bevorzugt ein Verhältnis von CO zu Wasserstoff ($H_2$) von 1:2 bzw. von $CO_2$ zu Wasserstoff ($H_2$) von 1:3 eingestellt. Für die Synthese von Methan wird bevorzugt ein Verhältnis von CO zu Wasserstoff ($H_2$) von 1 : 3 bzw. von $CO_2$ zu Wasserstoff ($H_2$) von 1 : 4 eingestellt.

In dem erfindungsgemäßen Verfahren wird die Synthese im Dreiphasenreaktor bevorzugt bei einer Betriebstemperatur von 200 bis < 350 °C, bevorzugter 220 bis 320 °C durchgeführt.

In einem weiteren Aspekt der Erfindung wird in dem erfindungsgemäßen Verfahren die Synthese im Dreiphasenreaktor bei einer Betriebstemperatur von.< 350 °C, bevorzugt ≤ 330 °C, ≤ 320 °C durchgeführt.

In einem weiteren Aspekt der Erfindung wird in dem erfindungsgemäßen Verfahren die Synthese im Dreiphasenreaktor bevorzugt bei einer Betriebstemperatur von ≥ 250 °C durchgeführt.

Dabei wird die Synthese von Methan bevorzugt bei einer Betriebstemperatur von 200 bis < 350 °C, bevorzugter 220 bis 330 °C, noch bevorzugter 250 bis 320 °C, noch bevorzugter 280 bis 320 °C durchgeführt.

Die Synthese von Methanol wird bevorzugt bei einer Betriebstemperatur von 220 bis 300 °C durchgeführt.

In einem weiteren Aspekt des erfindungsgemäßen Verfahrens erfolgt die Synthese im Dreiphasenreaktor bei einem Druck von 1 bis 100 bar (absolut), bevorzugt 5 bis 50 bar (absolut).

In einem weiteren Aspekt des erfindungsgemäßen Verfahrens erfolgt die Synthese von Methan bevorzugt bei einem Druck von 1 bis 50 bar (absolut), bevorzugt 5 bis 50 bar (absolut), bevorzugter 5 bis 20 bar (absolut).

In einem weiteren Aspekt des erfindungsgemäßen Verfahrens erfolgt die Synthese von Methan bei dem gleichen Druck, der in Zu- und Ableitungen und/oder in öffentlichen Gasnetzen herrscht.

In einem weiteren Aspekt des erfindungsgemäßen Verfahrens erfolgt die Synthese von Methanol bei einem Druck von 1 bis 100 bar (absolut), bevorzugt 10 bis 100 bar (absolut), bevorzugter 50 bis 100 bar (absolut).

Des Weiteren kann es vorteilhaft sein, in dem erfindungsgemäßen Verfahren Wasserdampf in den Dreiphasenreaktor einzuleiten, wobei eine Wasserdampfzugabe von 15 bis 30 %, bezogen auf den Eduktgasstoffmengenstrom, bevorzugt ist. Eine Wasserdampfzugabe erfolgt besonders bevorzugt bei der Synthese von Methan aus CO. Sofern eine Methanisierung von $CO_2$ ohne Anwesenheit von CO durchgeführt wird, ist eine Zugabe von Wasserdampf nicht erforderlich.

Dabei wird insbesondere für die Synthese von Methan bevorzugt ein Verhältnis von CO : $H_2$ von 1 : 3 bzw. von $CO_2$ : $H_2$ von 1 : 4 eingestellt. Das Verhältnis von CO : $CO_2$ ist darin von untergeordneter Bedeutung, solange ausreichend Wasserstoff für die jeweilige CO/$CO_2$ Komponente verfügbar ist, da die Umsetzung vorranging vom Wasserstoff-Gehalt abhängt. Außerdem ist für die Synthese von Methan eine Betriebstemperatur im Dreiphasenreaktor von 280 bis 350 °C bevorzugt. Desweiteren wird die Synthese von Methan bevorzugt bei einem Druck von 5 bis 20 bar durchgeführt. Bevorzugte Katalysatoren sind solche auf Ni-Basis, wobei bevorzugte Katalysatoren 65 - 75 Gew.% NiO und ein Bindemittel enthalten.

Entspricht das Verhältnis CO ($CO_2$) : $H_2$ nicht dem gewünschten Verhältnis, so kann nach der Vergasung der Biomasse bzw. vor der Umsetzung gemäß der vorliegenden Erfindung eine Wasser-Gas-Shift Reaktion durchgeführt werden (CO + $H_2O$ $\rightleftarrows$ $CO_2$ + $H_2$). Dabei ist jedoch darauf zu achten, dass nicht zu viel $CO_2$ für das vorhandene $H_2$ entsteht. Bevorzugt wird daher $H_2$ z.B. aus einer Elektrolyse zugesetzt werden (ohne vorherige Shift-Reaktion). Dabei wird dieser zusätzliche Wasserstoff bevorzugt ebenfalls an das erfindungsgemäß eingesetzte Wärmeträgermedium gebunden, um so in flüssiger Form mit den damit verbundenen Vorteilen in das Reaktionssystem eingebracht werden zu können.

Für die Synthese von Methanol wird insbesondere ein Verhältnis von CO : $H_2$ von 1 : 3 bzw. von $CO_2$ : $H_2$ von 1 : 4 eingestellt.

Durch die Verwendung eines hydrierten Wärmeträgermediums als Hydrierungsmittel ist es in dem erfindungsgemäßen Verfahren möglich und bevorzugt, die katalytische Füllung in direkten Kontakt mit dem Wasserstoff in Form des flüssigen hydrierten Wärmeträgermediums zu bringen.

Durch das erfindungsgemäße Verfahren, insbesondere auch durch die hierin beschriebenen Verfahrensoptimierungen, ist es möglich, Wasserstoff in hohen Konzentrationen für die Synthese direkt am Katalysator zur Verfügung zu stellen und das Gleichgewicht der Hydrierungsreaktion zuverlässig einzustellen. Im Synthesereaktor wird der Wasserstoff durch Dehydrierung des Wärmeträgermediums in aktivierter Form direkt am Katalysator für die Hydrierungsreaktion des kohlenstoffhaltigen Gases zur Verfügung gestellt. Dennoch kann die bei der exothermen Hydrierungsreaktion entstehende Wärme effizient aus dem System entfernt werden. Durch die endotherme Dehydrierung des Wärmeträgeröls im Synthesereaktor wird außerdem zusätzlich Wärme direkt am Katalysator abgeführt, was einen temperaturstabilen Betrieb des Reaktors weiter vereinfacht. Ferner sorgt die homogene Temperaturverteilung im Dreiphasensystem für eine hohe Langzeitstabilität der Katalysatoren. Im Gegensatz zum adiabaten Festbett ist eine Desaktivierung durch Sintern während der der Hydrierreaktion somit stark reduziert. Darüber hinaus ist die Hydrierung des Wärmeträgermediums aufgrund der hohen Energiedichte des Wärmeträgermediums (Wärmeträgeröls) auch ein effizientes Speichermedium für den fluktuierend anfallenden Wasserstoff.

Die vorliegende Erfindung umfasst desweiteren die Verwendung eines Reaktorsystems zur chemisch-katalytischen Synthese von Methan und/oder Methanol durch Hydrierung von CO und/oder $CO_2$ mit Wasserstoff, umfassend

a) einen (oder mehrere) Dreiphasenreaktor (a-a) der im Gegenstromverfahren betrieben wird und mit einer katalytischen Füllung beschickt ist,

b) eine Temperiervorrichtung (b-a) zur Temperierung eines hydrierten Wärmeträgermediums sowie gegebenenfalls eine Temperiervorrichtung (b-b) zur Temperierung des unhydrierten Wärmeträgermediums,

c) eine Vorrichtung zum Einleiten des temperierten (vor-)hydrierten Wärmeträgermediums in den Dreiphasenreaktor,

d) eine Vorrichtung zum Einleiten von CO und/oder $CO_2$ in den Dreiphasenreaktor und

e) eine Vorrichtung zum Abführen der umgesetzten Syntheseprodukte, sowie

f) einen dem Dreiphasenreaktor vorgeschalteten Reaktor (a-b) zur Hydrierung des Wärmeträgermediums, gegebenenfalls einen Speicher oder Vorratsbehälter (a-d) zur Aufnahme des unhydrierten Wärmeträgermediums zur Zuführung in den Hydrierungsreaktor (a-b) und/oder gegebenenfalls einen Speicher oder Vorratsbehälter (a-c) zur Aufnahme eines hydrierten Wärmeträgermediums, wobei ein solcher Speicher oder Vorratsbehälter (a-c) dem Dreiphasenreaktor (a-a) vorgeschaltet und dem gegebenenfalls vorgesehenen Hydrierungsreaktor (a-b) nachgeschaltet (und/oder nebengeschaltet) angeordnet ist,

g) gegebenenfalls eine Temperiervorrichtung zur Temperierung des CO und/oder $CO_2$,

h) gegebenenfalls eine Kreuzstromwärmeübertragung (h-a) für eine verbesserte Energieeffizienz des erfindungsgemäßen Verfahrens sowie

i) gegebenenfalls einen dem Dreiphasenreaktor vorgeschalteten Reaktor zur thermochemischen Vergasung von Biomasse zu CO- und/oder $CO_2$-haltigem Synthesegas,

zur Durchführung des erfindungsgemäßen Verfahrens.

Der Reaktor (a-b) zur (Vor-)Hydrierung des Wärmeträgermediums umfasst bevorzugt außerdem für die Hydrierung erforderliche Temperiereinrichtungen, Wärmeüberträger etc.

Der Reaktor (a-b) für die (Vor-)Hydrierung des Wärmeträgermediums ist in das erfindungsgemäße Reaktorsystem integriert bzw. mit diesem verbunden, um die kontinuierliche Verfahrensführung zu ermöglichen.

Das erfindungsgemäße Reaktorsystem (a-a) wird im Gegenstromverfahren betrieben.

Das erfindungsgemäße Reaktorsystem umfasst bevorzugt mindestens einen (oder mehrere) Dreiphasenreaktor(en) (a-a), ausgewählt aus der Gruppe umfassend Blasensäulenreaktoren und Rieselbettreaktoren (Trickle-Bed-Reaktor) oder Kombinationen davon, jeweils wie oben definiert, worin Rieselbettreaktoren (Trickle-Bed-Reaktoren) besonders bevorzugt sind.

Das erfindungsgemäße Reaktorsystem umfasst bevorzugt eine katalytische Füllung wie oben definiert.

In dem erfindungsgemäßen Reaktorsystem wird bevorzugt ein hydriertes bzw. hydrierbares Wärmeträgermedium eingesetzt, wie oben definiert.

Die in das erfindungsgemäße Reaktorsystem eingeleiteten kohlenstoffhaltigen Gase werden ausgewählt aus der Gruppe umfassend CO und $CO_2$.

In dem erfindungsgemäßen Reaktorsystem liegen bevorzugt die Synthesebedingungen mit Verhältnis von Kohlenstoffoxiden zu Wasserstoff (CO und/oder $CO_2$ zu $H_2$), Betriebstemperatur und Druck etc. vor, wie oben für das erfindungsgemäße Verfahren definiert.

Das erfindungsgemäße Reaktorsystem umfasst in einer weiteren Ausführungsform außerdem eine Vorrichtung zur Einleitung von Wasserdampf in den Dreiphasenreaktor.

## BESCHREIBUNG DER ABBILDUNGEN

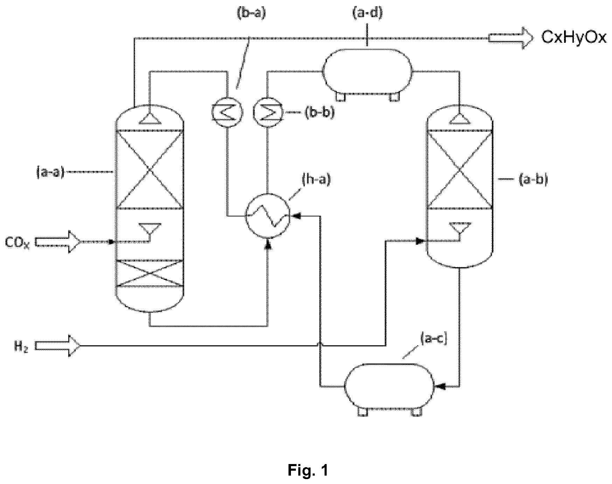

Fig. 1  Schematische Darstellung eines erfindungsgemäßen Reaktorsystems mit (a-a) Dreiphasenreaktor, (a-b) Reaktor zur Hydrierung des Wärmeträgermediums, (a-c) Speicher oder Vorratsbehälter für das (vor)hydrierte Wärmeträgermedium, (a-d) Speicher oder Vorratsbehälter für das unhydrierte Wärmeträgermedium, (b-a) Temperiervorrichtung zur Temperierung des (vor)hydrierten Wärmeträgermediums, (b-b) Temperiervorrichtung zur Temperierung des unhydrierten Wärmeträgermediums, (h-a) Kreuzstromwärmeübertragung (optional)

Figur 1 stellt lediglich eine schematische Darstellung eines erfindungsgemäßen Reaktorsystems dar. Die vorliegende Erfindung ist nicht auf die darin schematisch dargestellten Merkmale eingeschränkt zu interpretieren. Der Fachmann weiß, dass zusätzliche geeignete technische Vorrichtungen vorgesehen werden können, wie z.B. weitere Temperiervorrichtungen, Wärmeüberträger etc.. Solche zusätzlichen Temperiervorrichtungen oder Wärmeüberträger können z.B. insbesondere an der Zuleitung der Flüssigkeit in den Reaktor (a-b) zur Hydrierung des Wärmeträgermediums vorgesehen werden, selbst wenn in Figur 1 nicht explizit dargestellt. Derartige Vorrichtungen sind außerdem als optional zu betrachten und ein Fachmann ist in der Lage mit seinem Fachwissen eine geeignete Konfiguration des Reaktorsystems vorzunehmen.

Im Folgenden wird die Erfindung an Hand von Beispielen näher erläutert. Für den Fachmann ist ersichtlich, dass diese Beispiele nur exemplarisch sind und den Gegenstand der Erfindung nicht beschränken.

**BEISPIELE**

Versuchsbedingungen:

| | |
|---|---|
| Reaktor: | Autoklav benutzt als Dreiphasenreaktor |
| Katalysator: | kommerzieller $Ni/SiO_2$ Katalysator |
| Wärmeträgermedium: | PerhydroDibenzyltoluol von Hydrogenious Technologies GmbH |

Zum Nachweis der Transferhydrierung unter Methanisierungsbedingungen wurden experimentelle Versuche durchgeführt. 0,4 g von kommerziellem und reduziertem Katalysator wurde in 400 mL von Perhydrodibenzyltoluol (H18-DBT) suspendiert und in einem 1 L Edelstahlautoklavenreaktor unter inerter Atmosphäre (Argon) und atmosphärischem Druck auf 300 °C gemischt und geheizt. Unter diesen Bedingungen konnte $H_2$ im Gasstrom am Reaktoraustritt gemessen werden (siehe Beispiel 1). Dies ist ein Nachweis der Dehydrierung von H18-DBT.

**Referenzbeispiel 1 Dehydrierungsversuche**

| Nr. | T [°C] | y $H_2$ [%] | y $CH_4$ [%] |
|---|---|---|---|
| 1. | 200 | 0 | 0 |
| 2. | 250 | 0,37 | 0 |
| **3** | **300** | **3,61** | **1,11** |
| y = Stoffmengenanteil in der Gasphase<br>p = 1 bar (absolut) inerte Atmosphäre | | | |

Bei gleichen Bedingungen wurde danach 40 mL/min (NTP) $CO_2$ in den Reaktor dosiert. Die Analyse der Gaszusammensetzung am Reaktoraustritt konnte eine Reduktion des $H_2$-Stoffmengenstroms von 38 % mit einer $CO_2$-Reaktionsgeschwindigkeit zu $CH_4$ von 3,43 mmol pro s und pro kg Katalysator nachweisen (siehe Beispiel 2). Dieser Versuch zeigt die Methanerzeugung unter in-situ Bereitstellung von Wasserstoff aus LOHC.

**Referenzbeispiel 2 Methanisierungsversuchs**

Auf Basis des Dehydrierungsversuchs Nr. 3 aus Beispiel 1 wurden Methanisierungsversuche wie folgt durchgeführt:

| T [°C] | y $H_2$ [%] | r $CO_2$ [mmol/(kg s)] | C-Bilanz [%] |
|---|---|---|---|
| 300 | 0,52 | 2,88 | 98,59 |
| 350 | 6,22 | 6,20 | 112,61 |
| y = Stoffmengenanteil in der Gasphase <br> p = 1 bar (absolut), p $CO_2$ = 0,08 bar (absolut) <br> $T_{mod, CO2}$ = 20 kg/(s mol) | | | |

**Patentansprüche**

1. Verfahren zur Synthese von Methan ($CH_4$; SNG, Substitute Natural Gas) und/oder Methanol ($CH_3OH$) durch Hydrierung von CO und/oder $CO_2$ mit Wasserstoff, umfassend die folgenden Verfahrensschritte:

   a) Bereitstellen eines Reaktorsystems mit

      a-a) einem Dreiphasenreaktor, der im Gegenstromverfahren betrieben wird, sowie
      a-b) einem Reaktor zur Hydrierung eines Wärmeträgermediums und gegebenenfalls einem Speicher oder Vorratsbehälter für ein hydriertes Wärmeträgermedium (a-c) und/oder gegebenenfalls einem Speicher oder Vorratsbehälter für ein unhydriertes Wärmeträgermedium (a-d);

      b) Beschicken des Dreiphasenreaktors (a-a) mit einer katalytischen Füllung;
      c-a) Hydrierung des Wärmeträgermediums in dem Reaktor a-b),
      c) kontinuierliches Einleiten des hydrierten Wärmeträgermediums in den Dreiphasenreaktor (a-a) in Form einer flüssigen Phase, worin der Wasserstoff chemisch gebunden an das Wärmeträgermedium vorliegt;
      d) Einleiten von CO und/oder $CO_2$ in den Dreiphasenreaktor (a-a); und
      e) Abführen der umgesetzten Syntheseprodukte.

2. Verfahren nach Anspruch 1, worin der Dreiphasenreaktor a-a) ausgewählt wird aus der Gruppe umfassend Blasensäulenreaktoren und Rieselbettreaktoren (Trickle-Bed-Reaktor), worin Rieselbettreaktoren (Trickle-Bed-Reaktoren) bevorzugt sind.

3. Verfahren nach Anspruch 2, worin der Dreiphasenreaktor a-a) ausgewählt wird aus der Gruppe der Rieselbettreaktoren (Trickle-Bed-Reaktor).

4. Verfahren nach einem der vorhergehenden Ansprüche, worin das in Schritt c) eingeleitete hydrierte Wärmeträgermedium ein Liquid Organic Hydrogen Carrier (LOHC) der Formel LOHC-$H_n$ ist, worin n für einen ganzzahligen Wert von mindestens ($\geq$) 1 steht, wobei bevorzugt n $\geq$ 4 ist, und das ausgewählt wird aus der Gruppe umfassend Cykloalkene wie Dibenzyltoluol, Benzol, Toluol, Naphthalin, Biphenyl, Thiophen wie Methylthiophen, Carbazol wie N-Ethylcarbazol und Indol wie 2-Methylindol.

5. Verfahren nach einem der vorhergehenden Ansprüche, worin die katalytische Füllung gemäß Schritt b) ausgewählt ist aus der Gruppe umfassend Festkörperkatalysatoren, partikelförmige Katalysatoren, pulverförmige Katalysatoren, sowie Katalysatoren in Form katalytisch beschichteter Füllkörperpartikel, wobei katalytisch beschichtete Füllkörperpartikel bevorzugt sind, wobei bevorzugt ein Katalysator auf Basis von Metallen ausgewählt aus Fe, Co, Ni, Cu, Ru, Rh, Pd, Ag, Os, Ir, Pt, Au und Mo, sowie Kombinationen davon eingesetzt wird, wobei der Katalysator geträgert oder ungeträgert verwendet werden kann, wobei bevorzugte Trägermaterialien ausgewählt werden aus $Al_2O_3$, $SiO_2$, MgO und ZnO, sowie Kombinationen davon; besonders bevorzugt werden Katalysatoren auf Basis von Ni und Cu ausgewählt, wie insbesondere Katalysatoren auf Basis von NiO und Cu-ZnO-$Al_2O_3$ Systeme.

6. Verfahren nach Anspruch 5, wobei der Katalysator geträgert verwendet wird, wobei bevorzugte Trägermaterialien ausgewählt werden aus $Al_2O_3$, $SiO_2$, MgO und ZnO, sowie Kombinationen davon.

7. Verfahren nach Anspruch 5, wobei der Katalysator ausgewählt wird aus Katalysatoren auf Basis von NiO und Cu-ZnO-$Al_2O_3$ Systeme.

8. Verfahren nach einem der vorhergehenden Ansprüche, worin die Synthese im Dreiphasenreaktor bei einer Betriebstemperatur von 200 bis < 350 °C, bevorzugt 220 bis 320 °C erfolgt.

9. Verfahren nach einem der vorhergehenden Ansprüche, worin die Synthese im Dreiphasenreaktor bei einem Druck von 1 bis 100 bar (absolut), bevorzugt 5 bis 50 bar (absolut) erfolgt.

10. Verwendung eines Reaktorsystems zur chemisch-katalytischen Synthese von Methan und/oder Methanol durch Hydrierung von CO und/oder $CO_2$ mit Wasserstoff, umfassend

   a) einen Dreiphasenreaktor, der im Gegenstromverfahren betrieben wird und mit einer katalytischen Füllung beschickt ist,
   b) eine Temperiervorrichtung (b-a) zur Temperierung eines hydrierten Wärmeträgermediums sowie gegebenenfalls eine Temperiervorrichtung (b-b) zur Temperierung des unhydrierten Wärmeträgermediums,
   c) eine Vorrichtung zum Einleiten des temperierten hydrierten Wärmeträgermediums in den Dreiphasenreaktor,
   d) eine Vorrichtung zum Einleiten von CO und/oder $CO_2$ in den Dreiphasenreaktor und
   e) eine Vorrichtung zum Abführen der umgesetzten Syntheseprodukte, sowie
   f) einen dem Dreiphasenreaktor vorgeschalteten Reaktor (a-b) zur Hydrierung des Wärmeträgermediums, gegebenenfalls einen Speicher oder Vorratsbehälter (a-d) zur Aufnahme des unhydrierten Wärmeträgermediums und/oder gegebenenfalls einen Speicher oder Vorratsbehälter (a-c) zur Aufnahme eines hydrierten Wärmeträgermediums, wobei ein solcher Speicher dem Dreiphasenreaktor (a-a) vorgeschaltet und dem Hydrierungsreaktor (a-b) nachgeschaltet und/oder nebengeschaltet angeordnet ist,
   g) gegebenenfalls eine Temperiervorrichtung zur Temperierung des CO und/oder $CO_2$,
   h) gegebenenfalls eine Kreuzstromwärmeübertragung (h-a),
   zur Durchführung des Verfahrens gemäß einem der Ansprüche 1 bis 9.


**Claims**

1. A process for the synthesis of methane ($CH_4$; SNG, Substitute Natural Gas) and/or methanol ($CH_3OH$) by hydrogenation of CO and/or $CO_2$ with hydrogen, comprising following process steps:

   a) providing a reactor system with

      (a-a) a three-phase reactor operated in countercurrent process, as well as
      (a-b) a reactor for hydrogenation of a heat transfer medium and
      optionally a storage tank or reservoir for a hydrogenated heat transfer medium (a-c) and/or optionally a storage tank or reservoir for an unhydrogenated heat transfer medium (a-d);

   b) charging the three-phase reactor (a-a) with a catalytic charge;
   c-a) hydrogenation of the heat transfer medium in reactor (a-b),
   c) continuously introducing the hydrogenated heat transfer medium into the three-phase reactor (a-a) in the form of a liquid phase, wherein the hydrogen is chemically bound to the heat transfer medium;
   d) introducing CO and/or $CO_2$ into the three-phase reactor (a-a); and
   e) discharging the reacted synthesis products.

2. The process of claim 1, wherein the three-phase reactor (a-a) is selected from the group comprising bubble column reactors and trickle bed reactors, wherein trickle bed reactors are preferred.

3. The process according to claim 2, wherein the three-phase reactor (a-a) is selected from the group comprising trickle-bed reactors.

4. The process according to any one of the preceding claims, wherein the hydrogenated heat transfer medium introduced in step c) is a Liquid Organic Hydrogen Carrier (LOHC) of the formula LOHC-$H_n$, wherein n is an integer value of at least ($\geq$) 1, preferably $n \geq 4$, and which is selected from the group comprising cycloalkenes such as dibenzyltoluene, benzene, toluene, naphthalene, biphenyl, thiophene such as methylthiophene, carbazole such as N-ethylcarbazole and indole such as 2-methylindole.

5. The process according to any one of the preceding claims, wherein the catalytic charge according to step b) is selected from the group comprising solid catalysts, particulate catalysts, powdered catalysts, and catalysts in the form of catalytically coated packed particles, catalytically coated packed particles being preferred, wherein preferably a catalyst based on metals selected from Fe, Co, Ni, Cu, Ru, Rh, Pd, Ag, Os, Ir, Pt, Au and Mo, as well as combinations thereof is used, wherein the catalyst can be used supported or unsupported, wherein preferred support materials are selected from $Al_2O_3$, $SiO_2$, MgO and ZnO, as well as combinations thereof; particularly preferred are catalysts based on Ni and Cu, such as in particular catalysts based on NiO and $Cu$-$ZnO$-$Al_2O_3$ systems.

6.  The process according to claim 5, wherein a supported catalyst is used, wherein preferred support materials are selected from $Al_2O_3$, $SiO_2$, MgO and ZnO, as well as combinations thereof.

7.  The process of claim 5, wherein the catalyst is selected from catalysts based on NiO and $Cu$-$ZnO$-$Al_2O_3$ systems.

8.  The process according to any one of the preceding claims, wherein the synthesis in the three-phase reactor takes place at an operating temperature of 200 to < 350 °C, preferably 220 to 320 °C.

9.  The process according to any one of the preceding claims, wherein the synthesis in the three-phase reactor is carried out at a pressure of 1 to 100 bar (absolute), preferably 5 to 50 bar (absolute).

10. Use of a reactor system for the chemical-catalytic synthesis of methane and/or methanol by hydrogenation of CO and/or $CO_2$ with hydrogen, comprising

    a) a three-phase reactor which is operated in a countercurrent process and is charged with a catalytic charge,
    b) a temperature control device (b-a) for controlling the temperature of a hydrogenated heat transfer medium and optionally a temperature control device (b-b) for controlling the temperature of the unhydrogenated heat transfer medium,
    c) a device for supplying the tempered hydrogenated heat transfer medium to the three-phase reactor,
    d) a device for introducing CO and/or $CO_2$ into the three-phase reactor, and
    e) a device for discharging the reacted synthesis products, and
    f) a reactor (a-b) connected upstream of the three-phase reactor for hydrogenating the heat transfer medium, optionally a storage tank or reservoir (a-d) for receiving the unhydrogenated heat transfer medium and/or optionally a storage tank or reservoir (a-c) for receiving a hydrogenated heat transfer medium, such a storage tank being arranged upstream of the three-phase reactor (a-a) and downstream and/or alongside the hydrogenation reactor (a-b),
    g) optionally a temperature control device for controlling the temperature of the CO and/or $CO_2$,
    h) optionally a cross-flow heat transfer (h-a),
    for carrying out the process according to one of claims 1 to 9.

**Revendications**

1.  Procédé de synthèse de méthane ($CH_4$ ; GNS, Gaz Naturel de Synthèse) et/ou de méthanol ($CH_3OH$) par l'hydratation de CO et/ou de $CO_2$ avec de l'hydrogène, comprenant les étapes de procédé suivantes de :

    a) fournir un système de réacteur avec

    a-a) un réacteur à trois phases, qui fonctionne à contre-courant, ainsi que a-b) un réacteur pour l'hydratation d'un médium d'agent caloporteur et,
    le cas échéant, un récipient de stockage ou un réservoir d'un médium d'agent caloporteur hydrogéné (a-c) et/ou, le cas échéant, un récipient de stockage ou un réservoir d'un médium d'agent caloporteur non-hydrogéné (a-d) ;

    b) remplir le réacteur à trois phases (a-a) d'une substance catalytique ;
    c-a) hydrogéner le médium d'agent caloporteur dans le réacteur a-b),
    c) introduire de manière continue le médium d'agent caloporteur dans le réacteur à trois phases (a-a) en forme d'une phase liquide, dans laquelle l'hydrogène est présente de manière liée chimiquement au médium d'agent caloporteur ;
    d) introduire CO et/ou $CO_2$ dans le réacteur à trois phases (a-a) ; et
    e) évacuer les produits de synthèse obtenus.

**2.** Procédé selon la revendication 1, dans lequel le réacteur à trois phases a-a) est sélectionné dans le groupe comprenant des réacteurs à colonne à bulles et des réacteurs à lit à écoulement (trickle bed reactors), les réacteurs à lit à écoulement (trickle bed reactors) étant préférés.

**3.** Procédé selon la revendication 2, dans lequel le réacteur à trois phases a-a) est sélectionné dans le groupe des réacteurs à lit à écoulement (trickle bed reactors).

**4.** Procédé selon l'une des revendications précédentes, dans lequel le médium d'agent caloporteur hydrogéné introduit dans l'étape c) est un transporteur d'hydrogène organique liquide (LOHC) de la formule LOHC-H$_n$, dans laquelle n représente une valeur entière de minimum ($\geq$) 1, n étant de préférence $\geq$ 4, et il est sélectionné dans le groupe comprenant des cycloalcènes, tels que le dibenzyltoluène, le benzène, le toluène, le naphtalène, le biphényle, le thiophène, tel que le méthylthiophène, le carbazole, tel que le N-éthylcarbazole, et l'indole, tel que le 2-méthylindole.

**5.** Procédé selon l'une des revendications précédentes, dans lequel le remplissage catalytique selon l'étape b) est sélectionné dans le groupe comprenant des catalyseurs de corps solide, des catalyseurs en forme de particules, des catalyseurs en forme de poudre ainsi que des catalyseurs en forme de particules de matériau de remplissage à revêtement catalytique, les particules de matériau de remplissage à revêtement catalytique étant préférés, dans lequel un catalyseur à base des métaux sélectionné parmi Fe, Co, Ni, Cu, Ru, Rh, Pd, Ag, Os, Ir, Pt, Au et Mo ainsi que des combinaisons de ceux-cià une température de base est utilisé de préférence, dans lequel le catalyseur peut être utilisé de manière supportée ou non-supportée, des matériaux de support préférés étant sélectionnés parmi Al$_2$O$_3$, SiO$_2$, MgO et ZnO, ainsi que des combinaisons de ceux-ci ; des catalyseurs à base de Ni et de Cu, tels que notamment des catalyseurs à base de NiO et des systèmes de Cu-ZnO-Al$_2$O$_3$, sont sélectionnés de manière particulièrement préférée.

**6.** Procédé selon la revendication 5, dans lequel le catalyseur est utilisé de manière supportée, des matériaux de support préférés étant sélectionnés parmi Al$_2$O$_3$, SiO$_2$, MgO et ZnO ainsi que des combinaisons de ceux-ci.

**7.** Procédé selon la revendication 5, dans lequel le catalyseur est sélectionné parmi des catalyseurs à base de NiO et des systèmes de Cu-ZnO-Al$_2$O$_3$.

**8.** Procédé selon l'une des revendications précédentes, dans lequel la synthèse dans le réacteur à trois phases se fait à une température de base comprise entre 200 et < 350°C, de préférence entre 220 et 320°C.

**9.** Procédé selon l'une des revendications précédentes, dans lequel la synthèse dans le réacteur à trois phases se fait à une pression comprise entre 1 et 100 bar (absolu), de préférence entre 5 et 50 bar (absolu).

**10.** Utilisation d'un système de réacteur pour la synthèse chimique-catalytique de méthane et/ou de méthanol par l'hydratation de CO et/ou de CO$_2$ avec de l'hydrogène, comprenant :

    a) un réacteur à trois phases, qui fonctionne à contre-courant et qui est rempli d'un matériau catalytique,
    b) un dispositif de régulation de température (b-a) pour réguler la température d'un médium d'agent caloporteur hydrogéné ainsi que, le cas échéant, un dispositif de régulation de température (b-b) pour réguler la température d'un médium d'agent caloporteur non-hydrogéné,
    c) un dispositif d'introduction du médium d'agent caloporteur hydrogéné tempéré dans le réacteur à trois phases,
    d) un dispositif d'introduction de CO et/ou de CO$_2$ dans le réacteur à trois phases, et
    e) un dispositif destiné à évacuer les produits de synthèse obtenus, ainsi que
    f) un réacteur (a-b) disposé en amont du réacteur à trois phases pour l'hydratation du médium d'agent caloporteur, le cas échéant, un récipient de stockage ou un réservoir (a-d) du médium d'agent caloporteur non-hydrogéné et/ou, le cas échéant, un récipient de stockage ou un réservoir (a-c) pour recevoir un médium d'agent caloporteur hydrogéné, un tel récipient de stockage étant disposé en amont du réacteur à trois phases (a-a) et étant disposé en aval et/ou à côté du réacteur d'hydratation (a-b),
    g) si nécessaire, un dispositif de régulation de température pour tempérer le CO et/ou le CO$_2$,
    h) si nécessaire, une transmission de chaleur à flux croisé (h-a) pour effectuer le procédé selon l'une des revendications 1 à 9.

**Fig. 1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102011009163 A1
- CN 104645898 A
- CN 104549334 A
- WO 2014082801 A
- EP 1475349 A2


**In der Beschreibung aufgeführte Nicht-Patentliteratur**

- **GÖTZ et al.** Renewable Power-to-Gas: A technological and economic review. *Renewable Energy,* vol. 85, 1371-1390
- **VON HAGEN J.** Chemiereaktoren: Auslegung und Simulation. John Wiley & Sons, 2012, 10-10
- Three-Phase Trickle-Bed Reactors. **DUDUKOVIC et al.** Ullmann's Encyclopedia of Industrial Chemistry. 2013, 4